(19) **Europäisches Patentamt / European Patent Office / Office européen des brevets**

(11) **EP 4 470 451 A1**

(12) **EUROPEAN PATENT APPLICATION**

(43) Date of publication:
**04.12.2024 Bulletin 2024/49**

(21) Application number: **23176127.1**

(22) Date of filing: **30.05.2023**

(51) International Patent Classification (IPC):
**A61B 5/00** (2006.01)    **A61B 5/107** (2006.01)
**G06T 7/62** (2017.01)

(52) Cooperative Patent Classification (CPC):
**A61B 5/107; A61B 5/0064; G06T 7/62**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
- **Gutjahr, Ralf**
  **90459 Nürnberg (DE)**
- **Wimmer, Andreas**
  **91301 Forchheim (DE)**
- **Dr. Katzmann, Alexander**
  **90765 Fürth (DE)**

(74) Representative: **Siemens Healthineers Patent Attorneys**
**Postfach 22 16 34**
**80506 München (DE)**

(54) **A METHOD FOR ESTIMATING A BODY SURFACE AREA OF A SUBJECT AND MEDICAL IMAGING DEVICE**

(57)    A method and corresponding system of an imaging device for estimating a body surface area of a subject (5), the method comprising the following steps:(a) sampling the subject (5) with at least one sensor device (2) to acquire sample data (20) of the subject (5); (b) transferring the sample data (20) to a processing device (3); (c) on the processing device (3), applying at least one algorithm in order to determine the body surface area of the subject (5) based on the sample data (20).

FIG 8

EP 4 470 451 A1

**Description**

The invention relates to a method for estimating a body surface area of a subject such as a human being or animal, a corresponding computer program, a body surface estimation system, and a corresponding medical imaging device.

Independent of the grammatical term usage, individuals with male, female or other gender identities are included within the term.

Determining an appropriate drug dose for a patient for treatment and/or examination, e.g. for the application of a (chemo-)therapeutic agent or contrast media that may be used for medical imaging, is an important factor in medical workflows and patient outcome. A relatively simple and therefore convenient approach is a patient weight-based drug scaling. This approach can be particularly convenient because, for the determination of the drug-scaling, only the patient weight is needed. For this, the weight may be acquired from a hospital system such as a hospital information system (HIS) or a radiology information system (RIS) or by using a personal scale. Therefore, patient weight-based drug scaling is common practice in many clinical workflows. However, the drawback of this approach is a limited reliability and accuracy. For example, in particular when being based on information acquired from a hospital system, the weight information may be outdated and the patient's weight may have changed, e.g. due to illness effects. Furthermore, patient weight does not scale directly with the blood volume of the patient, while the blood volume is a determinant factor for drug scaling. Namely, this drug scaling based on patient weight does not consider that the respective drug traverses through the vascular system, visceral organs, and muscles to a greater extent than it does through body fat. Accordingly, the variances in body size and shape across different patients may not be taken into account by applying this weight-scaling based method. Particularly for obese patients this reduced contribution to dispersion and dilution of a drug can lead to overdosing, potentially resulting in an increased risk of impaired effectiveness of a therapy or suboptimal quality of image-based diagnosis, e.g. due to artifacts created due to overcon-trasting. An alternative to using the patient weight as basis for drug scaling, is to use the Body Mass Index (BMI) of the patient. However, similar effects can occur when a drug scal-ing is determined by using a patient's BMI, since the BMI may also be heavily be influence by the amount of body fat, and thus, the BMI is usually more a measure of body fat rather than a correlating measure of a patient's blood volume.

It has been found that linear drug scaling based on the patient's body surface area (BSA) can be more precise and reliable than drug scaling being based on patient weight or BMI, since the BSA tends to be about linear with respect to the blood volume. Furthermore, even beyond drug scaling, the body surface area (BSA) can be an important variable to be used for diagnosis and treatment planning in the medical context, for example in the case of skin burn.

However, manual calculation of the BSA can be time consuming and hinder a smooth workflow. Furthermore, objects that obstruct the view of the patient, such as clothes, respirators or tubes, may make it even more difficult to manually determine the BSA. For example, a systematic overestimate be a consequence. This may be especially critical in emergency situations, e.g. involving trauma. The BSA can be estimated using one of many different equations as proposed in early and recent literature. Most of these formulas rely on patient variables such as height, weight, and sometimes even sex and age. However, these formulas tend to be lacking additional variables indicative of physical or other characteristic variations, in particular concerning individual particularities of a patient, such as dwarfism, gigantism, anorexia, amputation. Furthermore, gathering the necessary variables for these formulas may still be a relatively laborious task.

It is therefore an object of the invention to provide a means to determine the body surface area in a more automatic and preferably more reliable way, preferably while being adaptable to individual patient properties.

This object is met or exceeded by a method according to claim 1, a computer program according to claim 13, a body surface estimation system according to claim 14, and a medical imaging device according to claim 15.

According to a first aspect of the invention, a method for estimating a body surface area of a subject, in particular a patient, is provided. The subject may, for example, be a human being or an animal. The method comprises the following steps:

(a) sampling the subject with at least one sensor device to acquire sample data, in particular three-dimensional sample data, of the subject;
(b) transferring the sample data to a processing device;
(c) on the processing device, applying at least one algorithm to the sample data in order to determine the body surface area of the subject based on the sample data.

Hence, the inventive concept is to use sample data of the subject that is acquired by the sensor device, in order to determine the body surface area (BSA) of the subject. The sensor device may advantageously be a sensor device that is already present at a clinical side, since sensor devices are often included in medical examination rooms. The sensor may be a three-dimensional (3D) sensor or a two-dimensional (2D) sensor. A 3D sensor is e.g. a 3D camera, whereas a 2D sensor may be an ordinary (2D) optical camera. On the other hand, it may be relatively easy and inexpensive to add such sensor device to the clinical site. Advantageously, the sampling of the subject via the sensor device may be performed automatically or semi-automatically, e.g. by manually activating the sensor device and then automatically taking and transferring the sample data. Accordingly, the effort for medical staff may be kept low. Furthermore, the BSA can be obtained quickly and reliably during a regular clinical workflow. Potentially, besides the steps of a usual clinical workflow, the subject does not even have to perform any additional actions, such as stepping on a scale, taking a certain pose or undress. Optionally, an additional step may be added to the method of planning a treatment and/or determining a drug scaling, such as contrast agent scaling, based on the determined body surface area. Optionally, the determined body surface area may be transferred to another device, such as an imaging device, in particular medical imaging device, a contrast injector, a hospital information system and/or a radiology information system. Imaging devices may, for example, be a magnetic resonance tomography system, a computed tomography system and/or an ultrasound system.

The sample data may, for example, be transferred to the processing device via a data cable or via a wireless connection. The processing device may be or may comprise, for example, a computer, such as a personal computer, a cloud computer, a server. Additionally, and/or alternatively, the processing device may be the processing unit of an imaging system. Advantageously, it has been found that the sample data can be enough information for the algorithm that is applied in order to determine a reliable and accurate estimation of the BSA. There are different alternatives of algorithms that may be applied. Corresponding embodiments are given below. Optionally, more than one algorithm may be applied. At least two algorithms may be applied consecutively or simultaneously. For example, it is conceivable that two algorithms are applied simultaneously and a further algorithm is applied afterwards or before. At least two different algorithms may determine the BSA essentially independently of each other. A further algorithm may compare the outcome of the two different algorithms or determine a BSA based on the outcome of the two different algorithms. Advantageously, an even greater reliability may be achieved by such a combination of independent algorithms.

The sample data may in particular comprise optical, acoustical, electromagnetic, topographic and/or tomographic data of the subject. Accordingly, the sensor device may, for example, be an optic sensor device, an acoustic sensor device, an electromagnetic sensor device, or a topographic sensor device. The sensor device is in particular configured to sample the subject's surface, typically a part of the subject's surface, in particular one side of the subject, namely the subject facing the sensor device. Typically, the subject will lie on a patient table, and the sensor device in configured to acquire sample data from above, capturing the upper side or part of the upper side of the subject. The sensor device may automatically take the sample data of the subject. For example, the sensor device may be configured to automatically take the sample data at a defined step of a clinical workflow. A defined step may for example comprise the patient lying on a patient table, such as a patient table of a medical imaging system, or the patient entering an examination room, e.g. through a door. Optionally, plurality of sensor devices may be used to independently acquire a plurality of sets of three-dimensional sample data. Advantageously, redundant information may be used to minimize an overall error due to individual accuracies of the sensor devices. In particular, complementary sample data may be fused together. Complementary sample data may, for example, be sample data measured from different angles with respect to the subject.

According to an embodiment, the at last one sensor device comprises an optical sensor, in particular a 3D camera, and/or a radar sensor and/or an acoustic sensor. The optical sensor may, for example, be an optical camera, in particular optical 3D camera, or a laser sampling device such as based on LiDAR (Light Detection and Ranging). The acoustic sensor may, for example, be based on ultrasound. A 3D camera may advantageously allow to directly acquire the three-dimensional sample data, in particular with a stationary sensor device. A sampling based on LiDAR may be performed by sending light pulses and detecting signals that are reflected by the subject. The runtime of the light may be used to calculate the distance to each part of the subject the light is reflected from. The light pulses may, for example be laser pulses or infrared light pulses. Preferably, based on the calculated distance, the shape of the subject is determined. The optical sensor may comprise a structured light scanner and be adapted to cast structured light on the subject. The structured light may in particular be a structured pattern, for example a grid, and the sensor device may be adapted to project the structured pattern on the subject. The structured light scanner may be configured to measure a distortion of the structured pattern that is projected on the subject. A position of different surface points of the subject may be calculated from the measured structured pattern on the subject, in particular in order to determine a shape of the subject.

According to an embodiment, the three-dimensional sample data is acquired by moving the sensor device or the subject, by triangulation, or by applying a time-of-flight measurement of the subject by the sensor device. In particular, the sensor device may comprise at least one two-dimensional sensor. The at least one two-dimensional sensor may be adapted to acquire three-dimensional sample data by moving into different positions relative to the subject and acquire position dependent sample data of the subject. The position-dependent sample data may be used to determine three-dimensional sample data. For performing triangulation, at least two sensor devices, in particular two-dimensional sensor devices, may be provided. The time-of-flight measurement may, for example be based on radar (radio detection and ranging) or LiDAR. The time-of-flight measurement may be based on sending radiation, in particular infrared light, and calculate the phase difference of reflected radiation.

According to an embodiment, a medical imaging device, in particular an X-ray based medical imaging device, is used as a or the sensor device that provides medical image data as sample data or as part of the sample data, in particular such that a pre-scan taken by the medical imaging device is used as medical image data. The pre-scan may for example be a topogram that is acquired before a main examination scan. The pre-scan may be a preliminary non-contrast scan. The X-ray-based imaging device may in particular be a computed tomography scanner. The sample data may, for example, be acquired from a topographic X-ray scan or a planar X-ray scan, for example in lateral and/or anterior-posterior direction. Advantageously, data that is to be measured anyway during a medical workflow may be used for the sample data. The acquisition of sample data may such be integrated into a medical workflow essentially seamlessly. Optionally, the sample data from the medical imaging device may be used to support sample data acquired with another sensor device. Hence, the sample data from the medical imaging device and another sensor device may be used as redundant information in order to minimize an overall error the data. The sample data from the medical imaging device may be used to adjust sample data acquired with another sensor device.

According to an embodiment, determining the body surface area of the subject comprises:

- creating a virtual avatar model of the subject's surface based on the sample data and a statistical shape model, such that the shape and pose of the avatar model is a fit to the sample data, wherein the fit adheres to boundary conditions defined by the statistical shape model,
wherein the statistical shape model comprises a database of a range of standard subject shapes and poses that define a range of shapes and poses the avatar model is bound to adhere to;
- calculating the surface area of the avatar model and using the calculated surface area of the avatar model as estimate for the body surface area.

The avatar model is in particular a model that describes the surface of the subject. The applied algorithm may in particular be configured to determine the avatar model such that the avatar model assumes both the pose and the body proportions of the observed subjects. In particular, the avatar model is determined such that it adheres to the statistical shape model. The statistical shape model may preferably be based on a database of body variations of real subjects, in particular real human beings or animals. For example, the avatar model may be determined as described by Singh, Vivek & Ma, Kai & Tamersoy, Birgi & Chang, Yao-Jen & Wimmer, Andreas & O'Donnell, Thomas and Chen, Terrence in "DARWIN: Deformable Patient Avatar Representation With Deep Image Network", 497-504, 10.1007/978-3-319-66185-8_56; 2017. The statistical shape model may preferably comprise shapes and positions that comprises a plurality of characteristic subject variations. Characteristic subject variations may comprise individual properties of different subjects, such as the subject being large or small. Individual properties may preferably comprise extreme properties such as including being extraordinarily small or large, being particularly thin or overweight and/or missing limbs. It has been found that the application of such an avatar model as intermediate step in order to calculate the surface area can lead to accurate and reliable results. Furthermore, the applied algorithm may only need a relatively low amount of training data, compared to a trained algorithm that is trained to directly determine a surface area, to achieve useful results of surface area determination. Furthermore, it has been found that the surface area can thus be determined quickly, in particular such that it can be integrated within a clinical workflow, usually without causing any detrimental delays of the workflow.

According to an embodiment, creating the virtual avatar model comprises allocating landmarks to at least some characteristic parts of the subject in the sample data, in particular by applying a first trained neural network, wherein the avatar model is created by mapping the avatar model to the landmarks, in particular by applying a second trained neural network. Characteristic parts may comprise defined parts of the head, such as the top and/or bottom of the head, shoulders, elbows, wrists, torso, such as the center of the torso, groin, knees and ankles. For example, 10 to 20 landmarks may be used. The first and/or second trained neural network may preferably be a convolutional neural network. The first/second neural network may in particular be configured and trained as described by Singh, Vivek & Ma, Kai & Tamersoy, Birgi & Chang, Yao-Jen & Wimmer, Andreas & O'Donnell, Thomas and Chen, Terrence in "DARWIN: Deformable Patient Avatar Representation With Deep Image Network", 497-504, 10.1007/978-3-319-66185-8_56; 2017.

According to an embodiment, the first trained neural network is trained to allocate landmarks despite the subject being at least partially covered by covering elements, in particular by clothes and/or other objects; wherein the first trained neural network is in particular trained such that the applied training data comprise at least some input sample data in which the subject is at least partially covered by the covering elements. Covering elements may in particular comprise clothes and/or blankets. Covering elements may also comprise objects such as oxygen tanks or parts of a medical imaging device. Hence, advantageously, this embodiment may allow to realistically estimate the surface area of the subject without the need for the subject to undress. An existing medical workflow may advantageously not have to be adapted, such as by removing a blanket or by undressing. This may save time during a workflow. Furthermore, not needing to undress may be particularly advantageous for persons or patients that are sick and/or weakened and may thus have trouble to undress. The first trained neural network may be trained with datasets comprising different subjects with different coverings. The training data may comprise sample data of subjects in different positions. The training data may preferably comprise sample data with and without coverings, in particular such that the training data comprise sample data, wherein a same subject in a same pose is comprised with and without cover.

According to an embodiment, after mapping the avatar model to the landmarks and before calculating the surface area, a model-fitting algorithm is applied such that the avatar model is adapted to better fit to the sample data within the boundaries defined by the statistical shape model, wherein the statistical shape model in particular comprises degrees of freedom allowing to modulate joints and a current orientation of joints of the subject. Thus, preferably, three algorithms or one algorithm with three parts, may be applied. The first algorithm of first part of the algorithm may define landmarks, the second algorithm or second part of the algorithm may perform a mapping of the avatar model to the landmarks, and the third algorithm or third part of the algorithm, i.e. the model-fitting algorithm, may adjust the avatar model. Optionally, the model-fitting algorithm may be configured to adhere to degrees of freedom within which joints of the avatar model may be modulated. Joints of the avatar model may, for example, comprise one, multiple or all of shoulders, elbows, wrists, knees and ankles of the subject.

According to an embodiment, the avatar model is or is represented by a three-dimensional geometric mesh of the body surface, in particular a mesh of triangles. The mesh can be well-suited to be used in order to calculate the surface. For example, the coordinates of individual elements of the mesh, in particular corner points, may be used to calculate the area of the individual elements. The area of the individual elements may be added up in order to calculate the full surface area of the subject. The elements of the mesh may in particular be the cells of the mesh. It has been found that a mesh of triangles can be particularly advantageous in order to calculate the surface area reliably and accurately. For example, the mesh may comprise a number N of triangles, $T_i$, each triangle being defined by three corner points, $v_{i,1}$, $v_{i,2}$, $v_{i,3}$. The corner points may be vectors defining the position of the corner points. The area of the elements of the mesh may be calculated by applying a vector product of coordinates defining the elements. The BSA may be calculated based on the areas of the elements, in particular by adding up the areas of the elements. For example, the individual area of a triangle $T_i$ may be calculated via the formula

$$\mathtt{A_i \; = \; 0,5 \; (v_{i,2} \; - \; v_{i,1}) \; \times \; (v_{i,3} \; - \; v_{i,1}),}$$

$\times$ being the vector product of the vectors. Accordingly, the surface area BSA of the avatar model may be determined via the calculation

$$BSA = \sum_{i=1}^{N} A_i$$

According to an embodiment, applying at least one algorithm comprises applying a convolutional neural network (CNN) that is trained to derive an estimate for the body surface area based on the sample data. This embodiment may in particular be useful, when a large amount of training data is available. For example, when a training database with an amount of data from subjects in the order of magnitude of hundred thousand is available, this embodiment can provide particularly reliable and robust results. Accordingly, for this embodiment, an avatar model does not have to be determined, but the surface area may be determined directly from the sample data. The CNN may be a residual convolutional neural network. For example, the CNN may comprise a series of residual blocks, each residual block consisting of a series of convolutional blocks. Each convolutional block may consist of a sequence of a convolution operator followed by an activation function, followed by a normalization function. Normalization layers may ensure an efficient training. Residual connections between convolutional blocks may ensure a sufficient gradient flow throughout the network. Each residual block may be followed by an average pooling operator. The CNN may preferably comprise a fully connected layer performing a body surface regression and producing a single output in the form of a body surface area. The output surface area may be a z-normalized BSA estimate.

The CNN may be trained via supervised training. The CNN may be trained by using paired data tuples, e.g. <x, y>. The data tuples may each comprise an input image, e.g. x, and a target variable, e.g. y. The input image may be derived from a sensor device, such as an optical or acoustical sensor. The target variable is in particular the surface area (BSA). Hence the target variable may be the ground truth of the training process. The BSA used for training may be measured or estimated by established formula, e.g. based on patient weight and height. Alternatively, the BSA of the target variables may be estimated based on a subject topogram. The subject topogram may provide an exact measurement of the patient surface. Alternatively, the target variable may be derived based on any of the embodiments employing the avatar model. Hence, the embodiment of the avatar model, requiring less training data, may be used to build up a training database for this embodiment. Some or all of the alternatives for acquiring target variables as described here may be combined, e.g. in order to acquire a larger amount of training data via different means. Advantageously, it has been found that, during training, the CNN may derive relevant criteria, such as defined by the statistical shape model according to other embodiments, automatically. Given a large enough amount of training data, the CNN may be able to outperform the quality of the training data, in particular by generalizing BSA factors other than patient weight and height, such as less common patient geometries or missing limbs.

According to an embodiment, prior to applying the convolutional neural network, an orthographic projection of the sample data is generated and the convolutional neural network is applied to the orthographic projection. It has been found that an orthographic projection may lead to more reliable results, since the input into the CNN may thus be independent on the specific positioning of the sensor device, in particular with regard to a distance between the sensor device and the subject. Hence, by applying the orthographic projection, the input data into the CNN may be standardized and the CNN thus may have to deal with a smaller range of different data. Preferably, the orthographic projection is always performed from essentially the same view angle with respect to the subject.

According to an embodiment, the body surface area is calculated based on the estimate of both the convolutional neural network and the estimate derived via the avatar model, in particular by determining an average or a weighted average of both estimates. Preferably, the two estimation methods may each be applied independently on the same sample data and the results may be combined afterwards. This combination may lead to more accurate and reliable results. A weighted average may in particular be applied when it is found that one of the two estimates tends to be more accurate. In this case the weighting may be such that more weight is put on the more accurate alternative. The weighting may be determined by further factors. For example, the weighting may depend on subject properties. This may, for example, be advantageous when one of the independent estimates is more reliable for some particular properties but less reliable for other particular properties.

According to an embodiment, the estimate of the body surface area derived from the convolutional neural network is compared to the estimate of the body surface area derived via the avatar model, wherein, in case that a difference in the estimated body surface area is found to be greater than a pre-defined threshold, a warning is sent to a user. Advantageously, two independently derived BSA may be used to make a reliability check. Hence, when the different of the two estimated BSA is greater than a threshold, it may be assumed that at least one estimate is faulty. It may then be advantageous to repeat the whole method, in particular with new sample data or to discard one of the estimates.

According to a further aspect of the invention, a computer program comprising instructions which, when the program is executed on a system comprising a processing device and at least one sensor, cause the system to carry out the steps of the method as described herein. All features and advantages of the method may be adapted to the computer program and vice versa.

According to a further aspect of the invention, a body surface estimation system is provided. The body surface estimation system comprises at least one sensor device, in particular a sensor device as described herein. The body surface estimation system further comprises a processing device configured to receive sensor signals from the at least one sensor device. Optionally, the body surface estimation system may comprise an output device configured to output derived information from the processing device. The processing device is configured to apply at least one algorithm in order to determine the body surface area of the subject based on the sample data. In particular, the body surface estimation system may be configured to carry out the method steps of the method for estimating a body surface area of a subject as described herein. All features and advantages of the method and of the body surface estimation system may be adapted to the computer program and vice versa.

According to a further aspect of the invention, a medical imaging device comprising a body surface estimation system as described herein is provided. All features and advantages of the method, of the body surface estimation system, and of the computer program may be adapted to the medical imaging device and vice versa.

The embodiments described herein may be combined with each other unless indicated otherwise.

The accompanying drawings illustrate various exemplary embodiments and methods of various aspects of the invention.

Fig. 1 shows a flow diagram of a method for estimating a body surface area of a subject according to an embodiment of the invention;

Fig. 2 shows a flow diagram of a method for estimating a body surface area of a subject according to another embodiment of the invention;

Fig. 3 shows a flow diagram of a method for estimating a body surface area of a subject according to another embodiment of the invention;

Fig. 4 shows a flow diagram of a method for estimating a body surface area of a subject according to another embodiment of the invention;

Fig. 5 shows a surface image reconstructed from three-dimensional sample data sampled with a 3D camera;

Fig. 6 shows an avatar model that is fitted to sample data with a method according to an embodiment of the invention;

Fig. 7 shows a zoom-in on the area marked in figure 6 by a dashed box;

Fig. 8 shows an avatar model according to an embodiment of the invention shown together with part of a reconstruction of sample data;

Fig. 9 shows the network architecture of a residual convolutional neural network according to an embodiment of the invention; and

Fig. 10 shows a medical imaging device comprising a body surface estimation system for estimating the body surface area of a subject according to an embodiment of the invention.

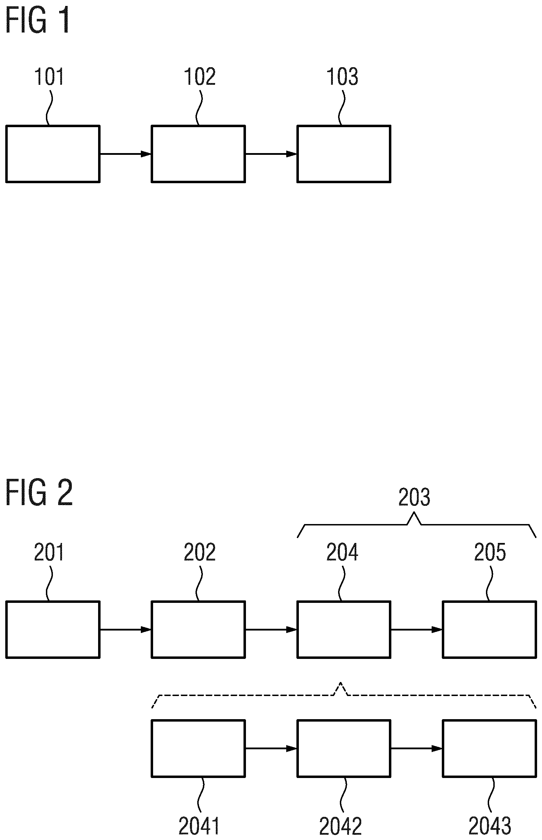

Figure 1 shows a flow diagram of a method for estimating a body surface area of a subject 5 according to an embodiment of the invention. In a first step 101, the subject 5 is sampled with at least one sensor device 2 to acquire three-dimensional sample data 20 of the subject 5. In this embodiment and in any other embodiment, the sensor device 2 may comprise an optical sensor such as a 3D camera and/or an acoustic sensor. Optionally, the sample data 20 may be acquired by triangulation, by moving the sensor device 2, or by applying a time-of-flight measurement of the subject 5 by the sensor device 2. Additionally or alternatively, the sample data 20 or some of the sample data 20 may be provided by a medical imaging device 1. Hence, the medical imaging device 1 may be used as sensor device 2. For example, a pre-scan performed by the medical imaging 1 device may be used as sample data 20. In a further step 102 the sample data 20 is transferred to a processing device 3. The processing device 3 may, for example, be part of a control unit of the medical imaging device 1. In a further step 103, on the processing device 3, at least one algorithm is applied in order to determine the body surface area of the subject 5 based on the sample data 20.

Figure 2 shows a flow diagram of a method for estimating a body surface area of a subject 5 according to another embodiment of the invention. In a first step 201, the subject 5 is sampled with at least one sensor device 2 to acquire three-dimensional sample data 20 of the subject 5. In a further step 202 the sample data 20 is transferred to a processing device 3. A further step 203 of applying at least one algorithm in order to determine the body surface area of the subject 5 based on the sample data 20 comprises two sub-steps 204, 205. Namely, a first sub-step 204 comprises creating a virtual avatar model of the subject's surface based on the sample data 20 and a statistical shape model. The statistical shape model comprises a database of a range of standard subject 5 shapes and poses that define a range of shapes and poses the avatar model is bound to adhere to. Accordingly, the avatar model is created such that the shape and pose of the avatar model is a fit to the sample data 20 that adheres to boundary conditions defined by the statistical shape model. Preferably, the avatar model may be a three-dimensional geometric mesh 11 of the body surface. Optionally, the first sub-step 204 may comprise consecutive steps 2041-2043. A first consecutive step 2041 of the consecutive steps 2041-2043 comprises allocating landmarks to at least some characteristic parts of the subject 5 in the sample data 20. A further consecutive step 2042 comprises mapping the avatar model to the landmarks in order to create the avatar model. Both, step 2041 and step 2042 may preferably each be performed by applying a neural network, in particular a convolutional neural network. Preferably, the trained neural network used for the step 2041 of allocating landmarks is trained to allocate landmarks despite the subject 5 being at least partially covered by covering elements such as clothes, blankets and/or other objects. In a further consecutive step 2043, performed after step 2042, a model-fitting algorithm is applied such that the avatar model is adapted to better fit to the sample data 20 within the boundaries defined by the statistical shape model. For this purpose, the statistical shape model may comprise degrees of freedom that allow to modulate joints and a current orientation of joints of the subject 5. In a further step 205, the surface area of the avatar model, in particular the adapted avatar model, is calculated and the calculated surface area of the avatar model is used as an estimate for the body surface area.

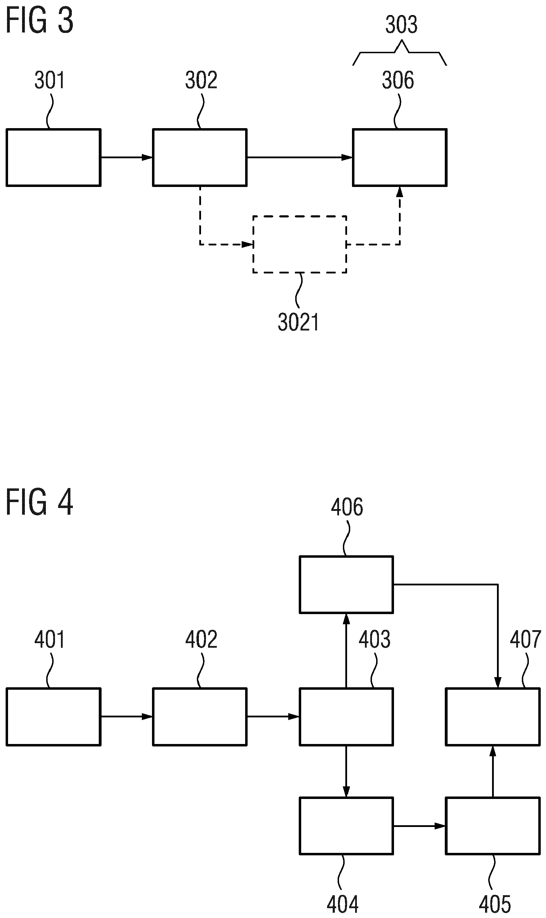

Figure 3 shows a flow diagram of a method for estimating a body surface area of a subject 5 according to an embodiment of the invention. In a first step 301, the subject 5 is sampled with at least one sensor device 2 to acquire three-dimensional sample data 20 of the subject 5. In a further step 302, the sample data 20 is transferred to a processing device 3. On the processing device 3, at least one algorithm is applied 303 in order to determine the body surface area of the subject 5 based on the sample data 20. The step of applying an algorithm comprises or consists of a step 306 of applying a convolutional neural network that is trained to derive an estimate for the body surface area based on the sample data 20. In an optional step 3021, prior to the step 306 of applying the convolutional neural network, an orthographic projection of the sample data 20 may be generated and during step 306, the convolutional neural network may be applied to the orthographic projection.

Figure 4 shows a flow diagram of a method for estimating a body surface area of a subject 5 according to an embodiment of the invention. In a first step 401, the subject 5 is sampled with at least one sensor device 2 to acquire three-dimensional sample data 20 of the subject 5. In a further step 402 the sample data 20 is transferred to a processing device 3. Optionally, in this step 402, the sample data 20 may be transferred to two different processing devices 3, in particular when the following steps 404 and 406 are performed on different processing devices 3. On the processing device 3 or on the two different processing devices 3, in a further step 403 at least two algorithms are applied to independently determine the body surface area of the subject 5 based on the sample data 20. In one step 406, a convolutional neural network is applied that is trained to derive an estimate for the body surface area based on the sample data 20. This step may in particular be performed as described with respect to figure 3, e.g. optionally including an orthographic projection of the sample data 20. In another step 404, an avatar model of the subject's surface based on the sample data 20 and a statistical shape model is created. The statistical shape model comprises a database of a range of standard subject shapes and poses that define a range of shapes and poses the avatar model is bound to adhere to. Accordingly, the avatar model is created such that the shape and pose of the avatar model is a fit to the sample data 20 that adheres to boundary conditions defined by the statistical shape model. In a further step 405, the surface area of the avatar model, in particular the adapted avatar model, is calculated and the calculated surface area of the avatar model is used as an estimate for the body surface area. These steps 404 and 405 may in particular be performed as described with respect to figure 2. In a further step 407, the body surface area is calculated based on the estimate of the route of step 404 and step 405 as well as the estimate of step 406. In particular, this step 407 may comprise determining an average or a weighted average of both estimates.

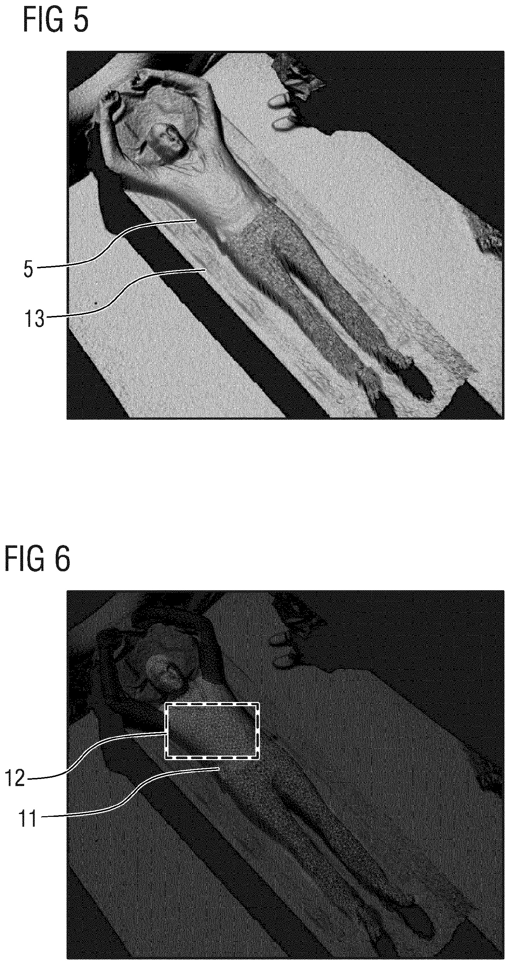

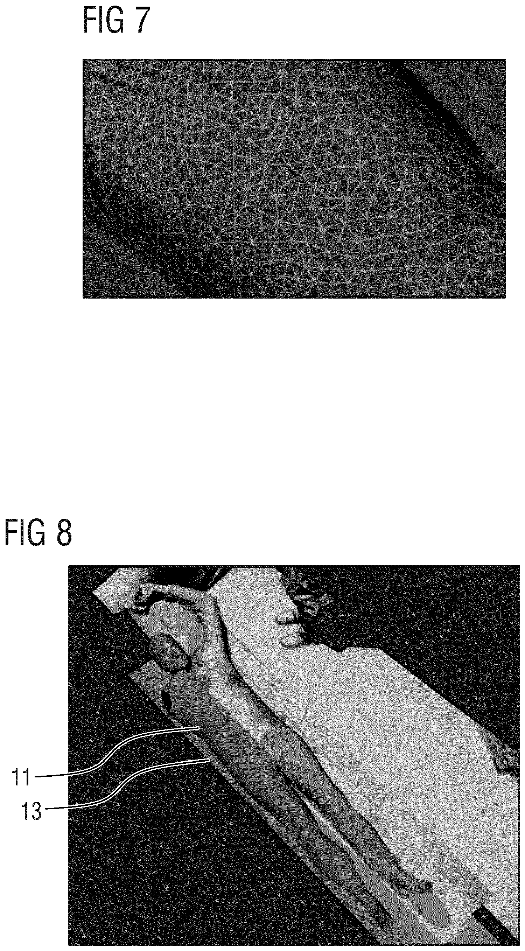

Figure 5 shows a surface image reconstructed from three-dimensional sample data 20 sampled with a 3D camera. On this image, a subject 5 can be seen lying on a patient table 13. In Figure 6, an avatar model is fitted to the sample data 20 with a method according to an embodiment of the invention. The avatar model comprises a three-dimensional geometric mesh 11 of the body surface. Figure 7 shows a zoom-in on the area marked in figure 6 by a dashed box 12. As can be seen, the geometric mesh 11 is a mesh of triangles. The area of the individual triangles may be calculated by via a vector product of the coordinates of the corners of the triangles. The full body surface area may then be calculated by adding the calculated areas of the individual triangles. Figure 8 shows the avatar model being shown together with part of the reconstruction of the sample data 20. As can be seen, the avatar model is fitted to the subject's body such that clothes of the subject 5 are actually placed above the avatar model. This shows that the avatar model as determined by an embodiment of the invention is not a fit to the outer clothes covering the subject 5 but to the surface of the actual subject 5. Hence, it can be seen that the method according to the invention is capable of accurately calculating the surface area of the subject 5, even though the subject 5 is partially covered with clothes. Furthermore, the avatar model follows the surface of the subject 5 above a patient table 13, thus being able to subtract the patient table 13 from the determination of the body surface area.

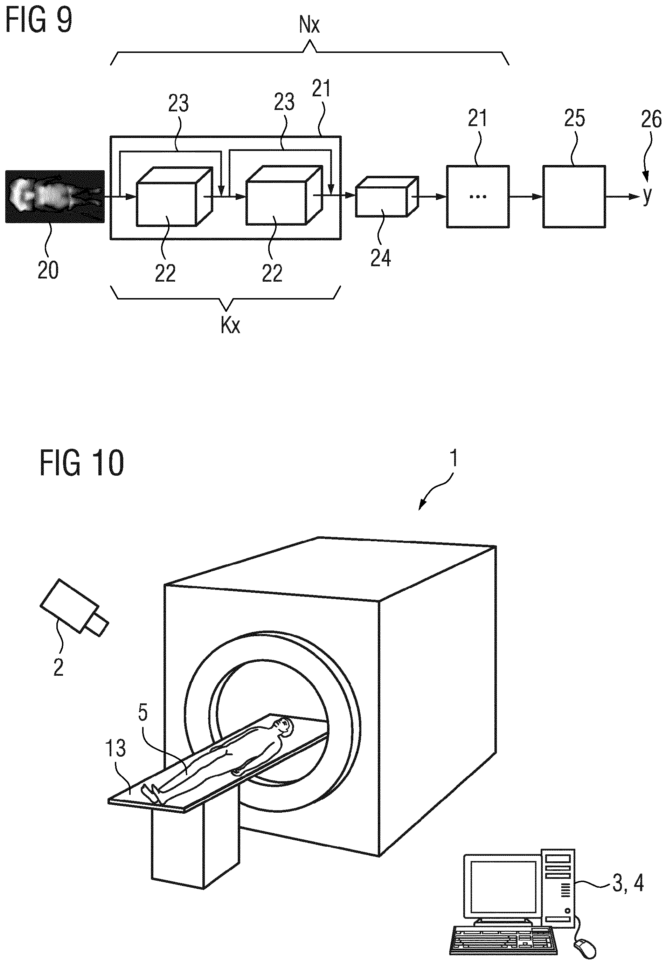

Figure 9 shows the network architecture of a residual convolutional neural network according to an embodiment of the invention. This convolutional neural network is configured to determine the body surface area of a subject 5 without the need of calculating a virtual avatar model based on three-dimensional sample data 20 of the subject 5. The network architecture consists of a series of N residual blocks 21 of K subsequent convolutional blocks 22 with residual connections 23. The residual connections 23 between convolutional blocks 22 may ensure a sufficient gradient flow throughout the network. Each convolutional block may consist of a sequence of a convolution operator followed by an activation function, followed by a normalization function. After each residual block, an average pooling operator 23 is applied (here shown only after the first residual block 23). The average pooling operator 23 reduces the image by half in each dimension (indicated by the smaller residual block 21 after the average pooling operator 23. Finally, a fully-connected layer 25 is appended, resulting in a BSA estimate 26 for the sample data 20 of the subject 5.

Figure 10 shows a medical imaging device 1 comprising a body surface estimation system for estimating the body surface area of a subject 5 according to an embodiment of the invention. The system comprises a sensor device 2 such as a 3D camera. The sensor device 2 is in particular configured to detect sample data 20 from the subject 5 lying on a patient table 13. The system further comprises a processing device 3 that is configured to receive sensor signals, in particular sensor signals transferring the sample data 20, from the sensor device 2. The processing device 3 is configured to apply at least one algorithm in order to determine/estimate the body surface area of the subject 5 based on the sample data 20. The estimation of the body surface area may, for example be carried out as described in any of the embodiments of figures 1 to 4. The system may also comprise an output device 4 that is configured to output information derived by the processing device 3.

## Claims

1. A method for estimating a body surface area of a subject (5), in particular a patient, the method comprising the following steps:

   (a) sampling the subject (5) with at least one sensor device (2) to acquire sample data (20), in particular three-dimensional sample data (20), of the subject (5);
   (b) transferring the sample data (20) to a processing device (3);
   (c) on the processing device (3), applying at least one algorithm to the sample data in order to determine the body surface area of the subject (5) based on the sample data (20).

2. The method according to claim 1,

   wherein determining the body surface area of the subject (5) comprises:

   - creating a virtual avatar model of the subject's surface based on the sample data (20) and a statistical shape model, such that the shape and pose of the avatar model is a fit to the sample data (20), wherein the fit adheres to boundary conditions defined by the statistical shape model,

   wherein the statistical shape model comprises a database of a range of standard subject shapes and poses that define a range of shapes and poses the avatar model is bound to adhere to;

   - calculating the surface area of the avatar model and using the calculated surface area of the avatar model as estimate for the body surface area.

3. The method according to claim 2,

   wherein creating the virtual avatar model comprises allocating landmarks to at least some characteristic parts of the subject (5) in the sample data (20), in particular by applying a first trained neural network, wherein the avatar model is created by mapping the avatar model to the landmarks, in particular by applying a second trained neural network.

4. The method according to claim 3,

   wherein the first trained neural network is trained to allocate landmarks despite the subject (5) being at least partially covered by covering elements, in particular by clothes and/or other objects; wherein the first trained neural network is in particular trained such that the applied training data comprise at least some input sample data (20) in which the subject (5) is at least partially covered by the covering elements.

5. The method according to claim 3 or 4, wherein, after mapping the avatar model to the landmarks and before calculating the surface area, a model-fitting algorithm is applied such that the avatar model is adapted to better fit to the sample data (20) within the boundaries defined by the statistical shape model, wherein the statistical shape model in particular comprises degrees of freedom allowing to modulate joints and a current orientation of joints of the subject (5).

6. The method according to any one of claims 2 to 5, wherein the avatar model is a three-dimensional geometric mesh (11) of the body surface, in particular a mesh of triangles.

7. The method according to any one of the preceding claims, wherein applying at least one algorithm comprises applying a convolutional neural network that is trained to derive an estimate for the body surface area based on the sample data (20).

8. The method according to claim 7, wherein, prior to applying the convolutional neural network, an orthographic projection of the sample data (20) is generated and the convolutional neural network is applied to the orthographic projection.

9. The method according to claim 7 or 8 and any one of claims 2 to 6, wherein the body surface area is calculated based on the estimate of both the convolutional neural network and the estimate derived via the avatar model, in particular by determining an average or a weighted average of both estimates.

10. The method according to any one of the preceding claims, wherein the at last one sensor device (2) comprises an optical sensor, in particular a 3D camera, and/or an acoustic sensor.

11. The method according to any one of the preceding claims, wherein three-dimensional sample data (20) is acquired by triangulation, by moving the sensor device (2) or the subject (5), or by applying a time-of-flight measurement of the subject (5) by the sensor device (2).

12. The method according to any one of the preceding claims, wherein a medical imaging device (1), in particular an X-ray based medical imaging device (1), is used as sensor device (2) that provides medical image data as sample data (20) or as part of the sample data (20), in particular such that a pre-scan taken by the medical imaging device (1) is used as medical image data.

13. A computer program comprising instructions which, when the program is executed on a system comprising a processing device (3) and at least one sensor, cause the system to carry out the steps of the method according to any one of the claims 1 to 12.

14. A body surface estimation system comprising at least one sensor device (2), in particular a sensor device (2) according to any one of claims 10-12,

   a processing device (3) configured to receive sensor signals from the at least one sensor device (2), and optionally an output device configured to output derived information from the processing device (3),
   wherein the processing device (3) is configured to apply at least one algorithm in order to determine the body surface area of the subject (5) based on the sample data (20),
   wherein the system is in particular configured to carry out the method steps of any one of claims 1-12.

15. A medical imaging device (1) comprising a body surface estimation system according to claim 14.

FIG 1

101 102 103

FIG 2

203

201 202 204 205

2041 2042 2043

## FIG 3

301

302

303

306

3021

## FIG 4

406

401

402

403

407

404

405

FIG 5

FIG 6

FIG 7

FIG 8

11

13

FIG 9

FIG 10

Europäisches
Patentamt
European
Patent Office
Office européen
des brevets

**EUROPEAN SEARCH REPORT**

Application Number

EP 23 17 6127

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | EP 3 742 397 A1 (KONINKLIJKE PHILIPS NV [NL]) 25 November 2020 (2020-11-25)<br>* paragraphs [0048] – [0051] *<br>* pages 97-120; figure 17 * | 1-6,10,<br>13-15<br><br>7-9,11,<br>12 | INV.<br>A61B5/00<br>A61B5/107<br>G06T7/62 |
| X | US 2022/230310 A1 (XIE LUKE [US] ET AL) 21 July 2022 (2022-07-21)<br>* paragraphs [0082] – [0084] *<br>* paragraphs [0119] – [0127] * | 1-9,<br>12-15 | |
| X<br><br><br><br><br><br><br><br><br><br><br><br>A | PICCIRILLI MARCO ET AL: "A Framework for Analyzing the Whole Body Surface Area from a Single View",<br>PLOS ONE,<br>vol. 12, no. 1,<br>3 January 2017 (2017-01-03), page e0166749, XP093032928,<br>DOI: 10.1371/journal.pone.0166749<br>Retrieved from the Internet:<br>URL:https://journals.plos.org/plosone/article/file?id=10.1371/journal.pone.0166749&type=printable><br>* pages 6-16 * | 1,2,10,<br>11,13-15<br><br><br><br><br><br><br><br><br><br><br><br>3-9,12 | |
| A | SINGH VIVEK ET AL: "DARWIN: Deformable Patient Avatar Representation With Deep Image Network",<br>4 September 2017 (2017-09-04), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 497 – 504, XP047429290,<br>ISBN: 978-3-540-74549-5<br>[retrieved on 2017-09-04]<br>* pages 498-501 * | 2-8,11 | |

TECHNICAL FIELDS
SEARCHED (IPC)

A61B
G06T

-/--

The present search report has been drawn up for all claims

| Place of search | Date of completion of the search | Examiner |
|---|---|---|
| Berlin | 20 October 2023 | Dydenko, Igor |

Europäisches
Patentamt
European
Patent Office
Office européen
des brevets

# EUROPEAN SEARCH REPORT

Application Number

EP 23 17 6127

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TIKUISIS P. ET AL: "Human body surface area: measurement and prediction using three dimensional body scans", EUROPEAN JOURNAL OF APPLIED PHYSIOLOGY, vol. 85, no. 3-4, 1 August 2001 (2001-08-01), pages 264-271, XP093093422, DE ISSN: 1439-6319, DOI: 10.1007/s004210100484 * the whole document * ----- | 1-15 | |

TECHNICAL FIELDS
SEARCHED       (IPC)

The present search report has been drawn up for all claims

| Place of search | Date of completion of the search | Examiner |
|---|---|---|
| Berlin | 20 October 2023 | Dydenko, Igor |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ....................................................................
& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 23 17 6127

This annex lists the patent family members relating to the patent documents cited in  the above-mentioned European search report.
The members are as contained in the European Patent Office EDP file on
The European Patent Office is in no way liable for these particulars which are merely given for the purpose of information.

20-10-2023

| Patent document cited in search report | | Publication date | Patent family member(s) | | Publication date |
|---|---|---|---|---|---|
| EP  3742397 | A1 | 25-11-2020 | BR | 112021023237 A2 | 04-01-2022 |
| | | | CN | 113874913 A | 31-12-2021 |
| | | | EP | 3742397 A1 | 25-11-2020 |
| | | | EP | 3973509 A1 | 30-03-2022 |
| | | | JP | 2022535703 A | 10-08-2022 |
| | | | KR | 20220013395 A | 04-02-2022 |
| | | | US | 2022301214 A1 | 22-09-2022 |
| | | | WO | 2020234339 A1 | 26-11-2020 |
| US 2022230310 | A1 | 21-07-2022 | CN | 114503159 A | 13-05-2022 |
| | | | EP | 4014201 A1 | 22-06-2022 |
| | | | JP | 2022544229 A | 17-10-2022 |
| | | | US | 2022230310 A1 | 21-07-2022 |
| | | | WO | 2021030629 A1 | 18-02-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SINGH, VIVEK** ; **MA, KAI** ; **TAMERSOY, BIRGI** ; **CHANG, YAO-JEN** ; **WIMMER, ANDREAS** ; **O'DON-NELL, THOMAS** ; **CHEN, TERRENCE**. *DARWIN: Deformable Patient Avatar Representation With Deep Image Network*, 2017, 497-504